# EUROPEAN PATENT APPLICATION

(11) **EP 4 206 201 A1**
(43) Date of publication of application: **05.07.2023**
(21) Application number: 21865949.8
(22) Date of filing: 06.09.2021
(51) Int. Cl.: C07D 471/04, A61K 31/519, A61P 35/00

(54) **CRYSTAL FORM OF AZETIDINE-SUBSTITUTED COMPOUND**

(30) Priority: 11.09.2020 CN 202010952692
(71) Applicant: Medshine Discovery Inc., Nanjing, Jiangsu 210032 (CN)
(72) Inventor: XU, Zhaobing, Shanghai 200131 (CN); ZHOU, Ming, Shanghai 200131 (CN); YAO, Ting, Shanghai 200131 (CN); HU, Lihong, Shanghai 200131 (CN); DING, Charles Z., Shanghai 200131 (CN); CHEN, Shuhui, Shanghai 200131 (CN)
(74) Representative: Impact Intellectual Property LLP
(86) International application number: PCT/CN2021/116756
(87) International publication number: WO 2022/052895

(57) **Abstract**

A crystal form of an azetidine-substituted compound as represented by formula (I) and a preparation method therefor. Further comprised is an application of the crystal form in preparing a drug for treating cancer.

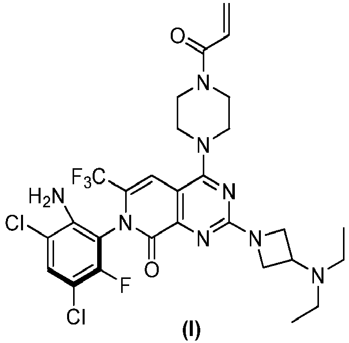

## Description

The present application claims priority to:
CN202010952692.5 filed on Sep. 11, 2020.

### TECHNICAL FIELD

The present invention relates to a crystal form of an azetidine-substituted compound and a method for preparing same, and also includes use of the crystal form in the preparation of a medicament for treating cancer.

### BACKGROUND

The first RAS oncogene was found in rat sarcoma, hence the name. RAS protein is a product of the expression of the RAS gene, and refers to a class of closely related monomer globulins consisting of 189 amino acids with a molecular weight of 21 KDa. It can bind to guanosine triphosphate (GTP) or guanosine diphosphate (GDP). The active state of the RAS protein has an influence on the growth, differentiation and skeleton of cells, the transportation and secretion of proteins, and the like. The activity of the RAS protein is regulated by binding to GTP or GDP. When binding to GDP, the RAS protein is in the dormant or "inactivated" state; when stimulated by specific upstream cell growth factors, the RAS protein is induced to exchange GDP and bind to GTP, which is referred to as the "activated" state. The RAS protein binding to GTP is able to activate downstream proteins for signaling. The RAS protein itself has weak hydrolysis activity for GTP, and is able to hydrolyze GTP to GDP. This allows the transition from the activated state to the inactivated state to be achieved. GAP (GTPase activating protein) is also required in this hydrolysis process. GAP can interact with the RAS protein to greatly promote its ability to hydrolyze GTP to GDP. Mutations in the RAS protein affect its interaction with GAP and thus its ability to hydrolyze GTP to GDP, keeping it in the activated state. The activated RAS protein continuously signals to downstream protein growth, which leads to the incessant growth and differentiation of cells, and ultimately to the occurrence of tumors. The RAS gene family is numerous in members, among which the subfamilies closely related to various cancers mainly include Kirsten rat sarcoma viral oncogene homolog (KRAS), Harvey rat sarcoma viral oncogene homolog (HRAS), and neuroblastoma rat sarcoma viral oncogene homolog (NRAS). It is found that approximately 30% of human tumors carry some mutations in the RAS gene, with KRAS mutations dominating by accounting for 86% of all RAS mutations. For KRAS mutations, the most common mutations occur at glycine 12 (G12), glycine 13 (G13) and glutamine 61 (Q61) residues, with the G12 mutation accounting for 83%.

The G12C mutation is a relatively common one of KRAS gene mutations, which refers to the mutation of glycine 12 to cysteine. The KRAS G12C mutation is most common in lung cancer, and occurs in about 10% of all lung cancer patients as extrapolated from data reported in the literature (Nat Rev Drug Discov 2014; 13: 828-851).

As a cutting-edge target, the KRAS G12C mutant protein has not been studied extensively. The literature (Nature. 2013; 503: 548-551) reports a class of KRAS G12C mutation-targeting covalent binding inhibitors. However, these compounds do not have high enzyme activity, nor do they show significant activity at the cellular level. The literature (Science 2016; 351: 604-608, Cancer Discov 2016; 6:316-29) reports a class of compounds that exhibit µM levels of cell antiproliferative activity at the cellular level. However, the metabolic stability of their structures is poor and it is also very difficult to further increase the activity. In addition to the few reports in the literature, Araxes Pharma LLC filed several patent applications on KRAS G12C inhibitors. For example, WO2016164675 and WO2016168540 report a class of quinazoline derivatives that have relatively high enzyme binding activity, show µM levels of cell antiproliferative activity, are structurally stable, and have certain selectivity. These compounds all have an acrylamide fragment that acts as a Michael addition acceptor and forms a covalent binding complex by reacting with the G12C residue on the KRAS protein. In 2018, Liu Yi et al. reported in Cell (Matthew R. Janes, Yi Liu et al., Cell, 2018, 172, 578-589) a KRAS G12C mutation-targeting covalent binding inhibitor, ARS-1620. The compound has very good metabolic stability, exhibits a nM level of cell antiproliferative activity at the cellular level, and can effectively inhibit tumor growth in a subcutaneous xenograft tumor model of the pancreatic cancer MIA-Paca2 cell. In the second half of 2018, Amgen Inc. started a phase I clinical recruitment (NCT03600883) for the KRAS G12C inhibitor AMG 510, which is the first organic small-molecule KRAS G12C inhibitor that has been clinically researched. At the AACR meeting in 2019, the structural formula and part of the preclinical research data of AMG 510 were disclosed. At the ASCO meeting in 2019, the early phase I clinical results of AMG 510 were published: the disease control rate of AMG 510 reached 90% in KRAS G12C mutation-containing non-small cell lung cancer patients who had achieved progressive disease after chemotherapy. In addition, a phase I clinical recruitment (NCT03785249) was also started in January 2019 for the KRAS G12C inhibitor MRTX849 developed by Mirati Therapeutics. Representative patents of the inhibitor include WO2017201161 and WO2019099524.

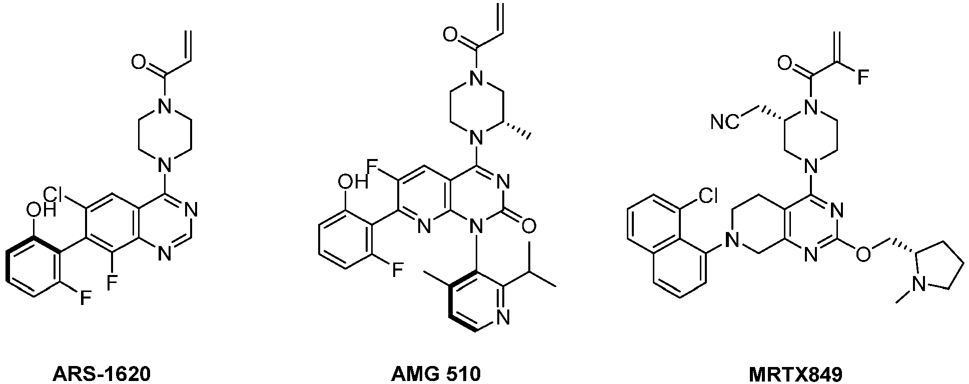

### SUMMARY

The present invention provides crystal form C of a compound of formula (I) 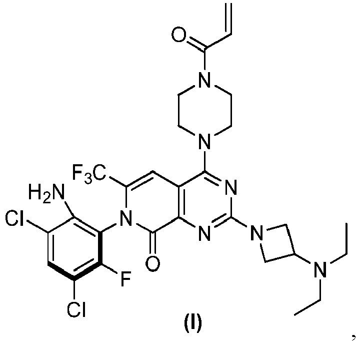 an X-ray powder diffraction pattern of which has characteristic diffraction peaks at the following 2Θ angles: 5.89±0.20°, 8.82±0.20°, and 17.71±0.20°.

In some embodiments of the present invention, the X-ray powder diffraction pattern of the crystal form C described above has characteristic diffraction peaks at the following 2Θ angles: 5.89±0.20°, 8.82±0.20°, 13.19±0.20°, 14.81±0.20°, 17.71±0.20°, 18.72±0.20°, 21.58±0.20°, and 24.47±0.20°.

In some embodiments of the present invention, the X-ray powder diffraction pattern of the crystal form C described above has characteristic diffraction peaks at the following 2Θ angles: 5.89°, 8.82°, 11.50°, 12.58°, 13.19°, 14.42°, 14.81°, 17.71°, 18.72°, 20.70°, 21.58°, 23.51°, 24.47°, 25.36°, 26.58°, 27.09°, and 29.08°. The present invention provides crystal form C of the compound of formula (I), the X-ray powder diffraction pattern of which has characteristic diffraction peaks at the following 2Θ angles: 5.89±0.20° and 8.82±0.20°, and has characteristic peaks at 17.71±0.20°, and/or 11.50±0.20°, and/or 12.58±0.20°, and/or 13.19±0.20°, and/or 14.42±0.20°, and/or 14.81±0.20°, and/or 18.72±0.20°, and/or 20.70±0.20°, and/or 21.58±0.20°, and/or 23.51±0.20°, and/or 24.47±0.20°, and/or 25.36±0.20°, and/or 26.58±0.20°, and/or 27.09±0.20°, and/or 29.08±0.20°.

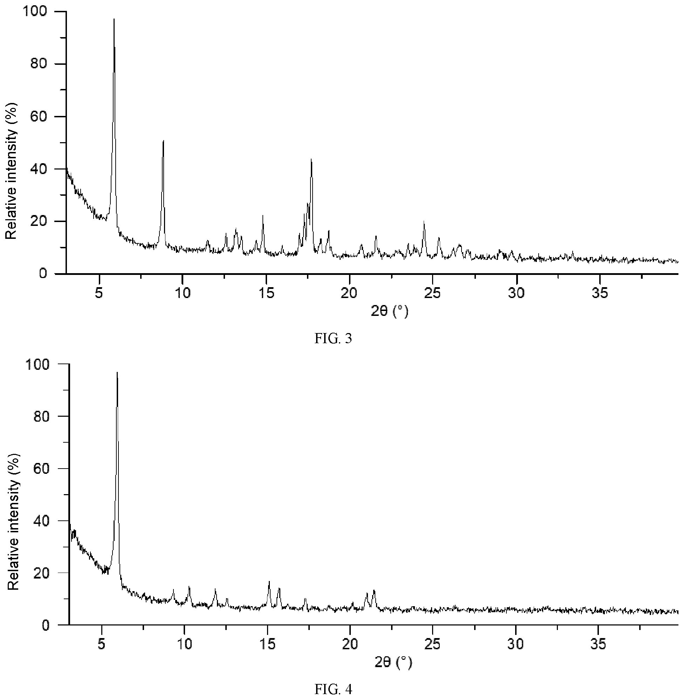

In some embodiments of the present invention, the XRPD pattern of the crystal form C described above is as shown in FIG. 3.

In some embodiments of the present invention, analytical data of the XRPD pattern of the crystal form C described above are as shown in Table 3.

**Table 3. Analytical data of the XRPD pattern of crystal form C**

| **No.** | **2θ angle (°)** | **Interplanar spacing (Å)** | **Relative intensity (%)** |
|---|---|---|---|
| 1 | 5.89 | 14.99 | 100.00 |
| 2 | 8.82 | 10.02 | 52.74 |
| 3 | 11.50 | 7.69 | 5.15 |
| 4 | 12.58 | 7.04 | 8.22 |
| 5 | 13.19 | 6.71 | 10.34 |
| 6 | 14.42 | 6.14 | 5.69 |
| 7 | 14.81 | 5.98 | 16.80 |
| 8 | 17.71 | 5.01 | 45.95 |
| 9 | 18.72 | 4.74 | 11.23 |
| 10 | 20.70 | 4.29 | 5.78 |
| 11 | 21.58 | 4.12 | 9.97 |
| 12 | 23.51 | 3.78 | 6.46 |
| 13 | 24.47 | 3.64 | 16.99 |
| 14 | 25.36 | 3.51 | 9.10 |
| 15 | 26.58 | 3.35 | 5.90 |
| 16 | 27.09 | 3.29 | 3.43 |
| 17 | 29.08 | 3.07 | 3.11 |

In some embodiments of the present invention, a differential scanning calorimetry curve of the crystal form C described above has an endothermic peak starting point at 214.7±5 °C.

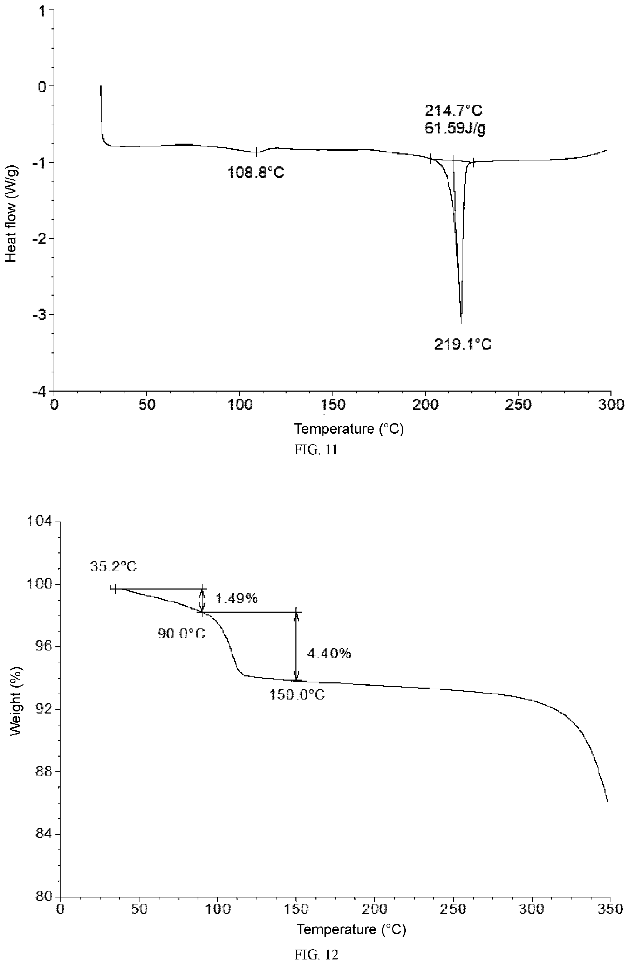

In some embodiments of the present invention, a DSC profile of the crystal form C described above is as shown in FIG. 11.

In some embodiments of the present invention, a thermogravimetric analysis curve of the crystal form C described above shows a weight loss of 2.52% at 150.0±3 °C.

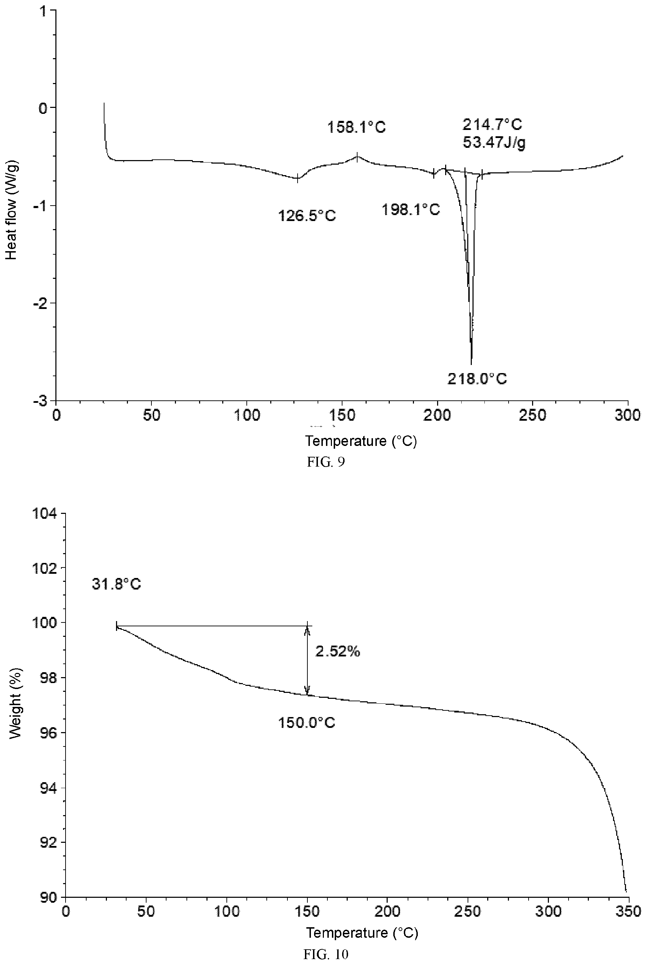

In some embodiments of the present invention, a TGA profile of the crystal form C described above is as shown in FIG. 10.

The present invention provides crystal form A of the compound of formula (I), analytical data of an XRPD pattern of which are as shown in Table 1.

**Table 1. Analytical data of an XRPD pattern of crystal form A**

| **No.** | **2θ angle (°)** | **Interplanar spacing (Å)** | **Relative intensity (%)** |
|---|---|---|---|
| 1 | 5.99 | 14.77 | 100.00 |
| 2 | 6.22 | 14.20 | 49.11 |
| 3 | 6.68 | 13.22 | 10.34 |
| 4 | 8.45 | 10.46 | 16.16 |
| 5 | 9.09 | 9.73 | 3.23 |
| 6 | 10.90 | 8.12 | 7.21 |
| 7 | 12.43 | 7.12 | 9.56 |
| 8 | 13.36 | 6.63 | 5.38 |
| 9 | 14.82 | 5.98 | 1.77 |
| 10 | 16.18 | 5.48 | 5.08 |
| 11 | 17.34 | 5.12 | 1.51 |
| 12 | 18.25 | 4.86 | 11.59 |
| 13 | 18.90 | 4.70 | 8.20 |
| 14 | 19.90 | 4.46 | 4.26 |
| 15 | 21.00 | 4.23 | 4.63 |
| 16 | 21.85 | 4.07 | 4.64 |
| 17 | 22.65 | 3.93 | 5.36 |
| 18 | 24.88 | 3.58 | 3.97 |

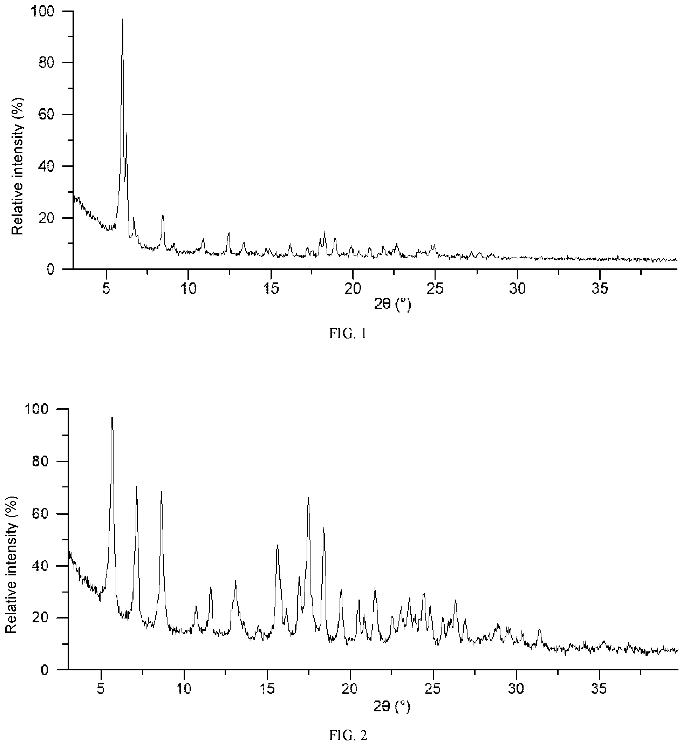

The present invention provides crystal form A of the compound of formula (I), the XRPD pattern of which is as shown in FIG. 1.

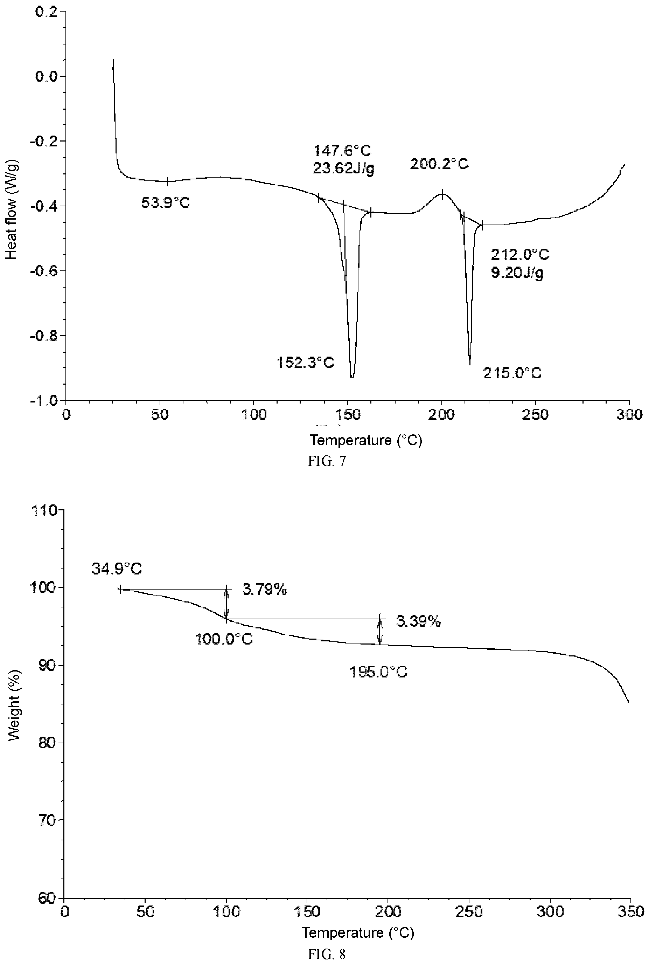

In some embodiments of the present invention, a DSC profile of the crystal form A described above is as shown in FIG. 7.

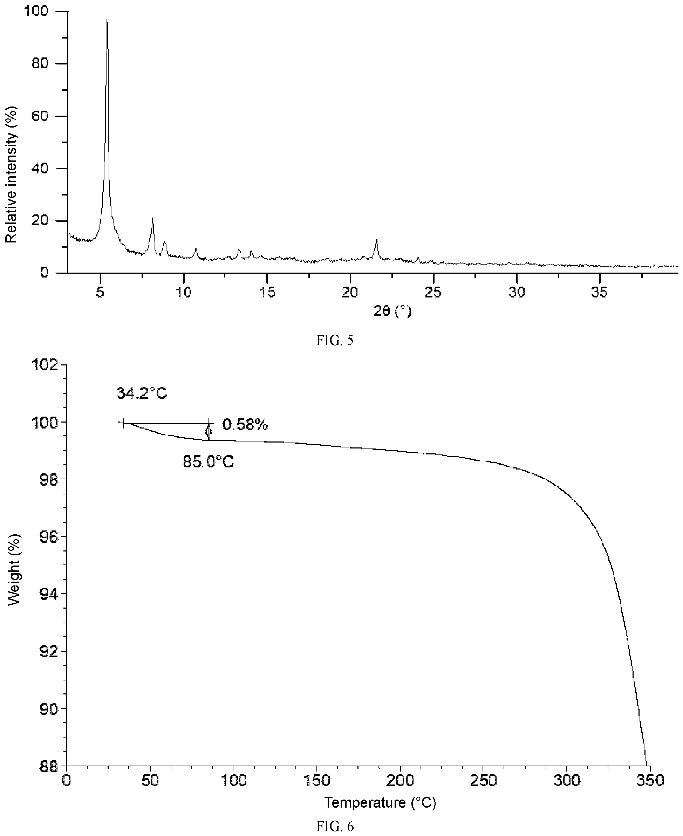

In some embodiments of the present invention, a TGA profile of the crystal form A described above is as shown in FIG. 6.

The present invention provides crystal form B of the compound of formula (I), analytical data of an XRPD pattern of which are as shown in Table 2.

**Table 2. Analytical data of an XRPD pattern of crystal form B**

| **No.** | **2θ angle (°)** | **Interplanar spacing (Å)** | **Relative intensity (%)** |
|---|---|---|---|
| 1 | 5.66 | 15.61 | 100.00 |
| 2 | 7.15 | 12.37 | 70.05 |
| 3 | 8.65 | 10.22 | 66.59 |
| 4 | 10.71 | 8.26 | 12.81 |
| 5 | 11.59 | 7.63 | 24.49 |
| 6 | 13.11 | 6.75 | 26.88 |
| 7 | 14.47 | 6.12 | 4.31 |
| 8 | 15.63 | 5.67 | 48.15 |
| 9 | 16.16 | 5.48 | 12.93 |
| 10 | 16.93 | 5.24 | 32.01 |
| 11 | 17.49 | 5.07 | 72.73 |
| 12 | 18.41 | 4.82 | 58.45 |
| 13 | 19.45 | 4.56 | 26.29 |
| 14 | 20.50 | 4.33 | 20.49 |
| 15 | 20.85 | 4.26 | 11.86 |
| 16 | 21.49 | 4.14 | 27.34 |
| 17 | 22.53 | 3.95 | 13.25 |
| 18 | 23.06 | 3.86 | 17.45 |
| 19 | 23.55 | 3.78 | 21.51 |
| 20 | 24.42 | 3.65 | 25.70 |
| 21 | 24.80 | 3.59 | 18.99 |
| 22 | 25.56 | 3.48 | 13.75 |
| 23 | 26.34 | 3.38 | 22.68 |
| 24 | 26.93 | 3.31 | 13.73 |
| 25 | 28.91 | 3.09 | 11.60 |
| 26 | 29.54 | 3.02 | 7.99 |
| 27 | 30.35 | 2.94 | 7.33 |
| 28 | 31.40 | 2.85 | 10.21 |

The present invention provides crystal form B of the compound of formula (I), the XRPD pattern of which is as shown in FIG. 2.

In some embodiments of the present invention, a DSC profile of the crystal form B described above is as shown in FIG. 9.

In some embodiments of the present invention, a TGA profile of the crystal form B described above is as shown in FIG. 8.

The present invention provides crystal form D of the compound of formula (I), analytical data of an XRPD pattern of which are as shown in Table 4.

**Table 4. Analytical data of an XRPD pattern of crystal form D**

| **No.** | **2θ angle (°)** | **Interplanar spacing (Å)** | **Relative intensity (%)** |
|---|---|---|---|
| 1 | 5.93 | 14.91 | 100.00 |
| 2 | 9.29 | 9.52 | 5.05 |
| 3 | 10.27 | 8.61 | 8.78 |
| 4 | 11.86 | 7.47 | 8.08 |
| 5 | 15.10 | 5.87 | 12.81 |
| 6 | 15.70 | 5.65 | 10.04 |
| 7 | 17.28 | 5.13 | 5.43 |
| 8 | 21.00 | 4.23 | 6.91 |
| 9 | 21.44 | 4.15 | 8.13 |

The present invention provides crystal form D of the compound of formula (I), the XRPD pattern of which is as shown in FIG. 4.

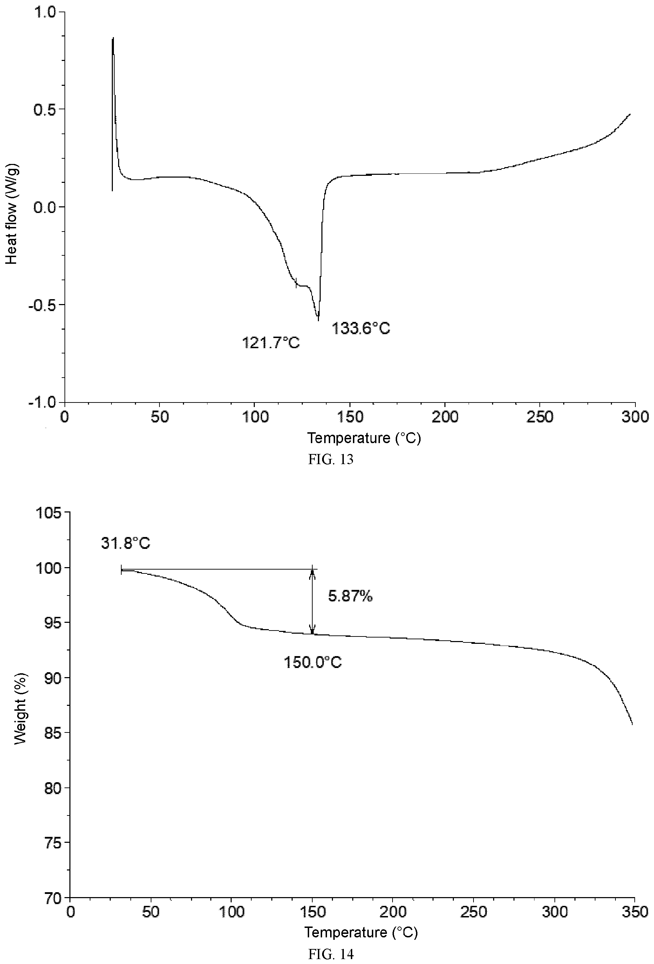

In some embodiments of the present invention, a DSC profile of the crystal form D described above is as shown in FIG. 13.

In some embodiments of the present invention, a TGA profile of the crystal form D described above is as shown in FIG. 12.

The present invention provides crystal form E of the compound of formula (I), analytical data of an XRPD pattern of which are as shown in Table 5.

**Table 5. Analytical data of an XRPD pattern of crystal form E**

| **No.** | **2θ angle (°)** | **Interplanar spacing (Å)** | **Relative intensity (%)** |
|---|---|---|---|
| 1 | 5.38 | 16.42 | 100.00 |
| 2 | 8.11 | 10.91 | 16.21 |
| 3 | 8.86 | 9.99 | 5.94 |
| 4 | 10.74 | 8.24 | 4.14 |
| 5 | 13.30 | 6.66 | 4.33 |
| 6 | 14.07 | 6.30 | 3.26 |
| 7 | 21.57 | 4.12 | 9.51 |
| 8 | 24.05 | 3.70 | 2.20 |
| 9 | 29.58 | 3.02 | 0.92 |
| 10 | 30.72 | 2.91 | 0.84 |

The present invention provides crystal form E of the compound of formula (I), the XRPD pattern of which is as shown in FIG. 5.

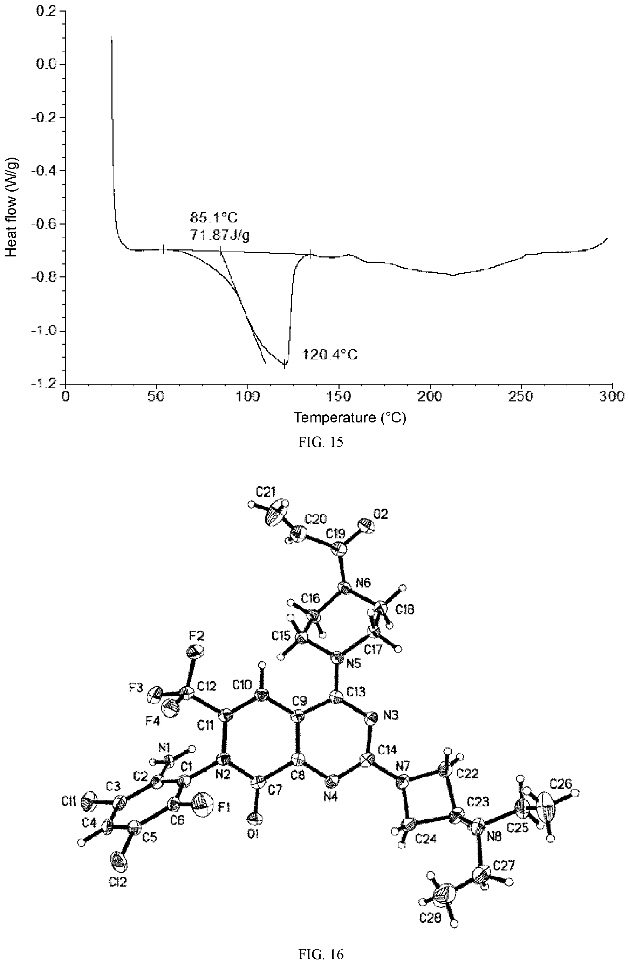

In some embodiments of the present invention, a DSC profile of the crystal form E described above is as shown in FIG. 15.

In some embodiments of the present invention, a TGA profile of the crystal form E described above is as shown in FIG. 14.

In some embodiments of the present invention, use of the above crystal forms in the preparation of a medicament for treating cancer is provided.

In some embodiments of the present invention, the cancer described above includes lung cancer, lymphoma, esophageal cancer, ovarian cancer, pancreatic cancer, rectal cancer, brain glioma, cervical cancer, urothelial cancer, stomach cancer, endometrial cancer, liver cancer, bile duct cancer, breast cancer, colon cancer, leukemia, and melanoma.

### Technical Effects

The compound of the present invention has good cellular activity and selectivity, and its crystal forms are stable and have good solubility, proper hygroscopicity, and good druggability.

### Definitions and Description

The present invention uses the following abbreviations: DMSO for dimethyl sulfoxide; MeOH for methanol; TFA for trifluoroacetic acid; NCS for N-chlorosuccinimide; PyBrOP for bromo-trispyrrolidinophosphonium hexafluorophosphate; LCMS for high performance liquid chromatography-mass spectrometry; XRPD for X-ray powder diffraction; HPLC for high performance liquid chromatography; ACN for acetonitrile; and H₂O for water.

### XRPD test parameters

**Table 6. XRPD test parameters**

| Parameter | **XRPD** (reflection mode) | **XRPD** (varying-temperature mode) | **XRPD** (reflection mode) |
|---|---|---|---|
| Model | Empyrean | Empyrean | X'Pert3 |
| X-ray | Cu, ka, Kα1 (Å): 1.540598; Ka2 (Å): 1.544426; | Cu, ka, Kα1 (Å): 1.540598; Ka2 (Å): 1.544426; | Cu, ka, Kα1 (Å): 1.540598; Ka2 (Å): 1.544426; |
| | Intensity ratio Kα2/Kα1: 0.50 | Intensity ratio Kα2/Kα1: 0.50 | Intensity ratio Kα2/Kα1: 0.50 |
| X-ray light tube settings | 45 kV, 40mA | 45 kV, 40mA | 45 kV, 40mA |
| Divergent slit | Fixed 1/8° | Fixed 1/8° | Fixed 1/8° |
| Scan mode | Continuous | Continuous | Continuous |
| Scan range (°2Theta) | 3∼40 | 3∼40 | 3∼40 |
| Length of each scan step (s) | 17.8 | 33.0 | 46.7 |
| Scan step size | 0.0167 | 0.0167 | 0.0263 |

### Thermogravimetric analysis (TGA) and differential scanning calorimetry (DSC)

TGA and DSC profiles were recorded on a TA Discovery TGA5500/Q5000 thermogravimetric analyzer and a TAQ200/Q2000/Discovery DSC 2500 differential scanning calorimeter, respectively. The test parameters are listed in Table 7.

**Table 7**

| Parameter | **TGA** | **DSC** |
|---|---|---|
| Method | Linear heating | Linear heating |
| Sample tray | Aluminum tray, open | Aluminum tray, closed |
| Temperature range | Room temperature-set endpoint temperature | Room temperature/25 °C-set endpoint temperature |
| Scan speed (°C/min) | 10 | 10 |
| Protective gas | Nitrogen | Nitrogen |

### High performance liquid chromatography (HPLC)

The purity and solubility of a sample are determined through a high performance liquid chromatograph Agilent 1260 HPLC.

**Table 8-1. HPLC test parameters for purity**

| **Parameter** | **Set value** | |
|---|---|---|
| Chromatography column | SymmetryShield RP18, 150 × 4.6 mm, 3.5 µm | |
| Mobile phase | A: 0.1% TFA in water | |
| | B: ACN | |
| Gradient | Time (min) | %Mobile phase B |
| | 0.0 | 25 |
| | 2.0 | 25 |
| | 32.0 | 50 |
| | 35.0 | 90 |
| | 40.0 | 90 |
| | 41.0 | 25 |
| | 51.0 | 25 |
| Time | 51 min | |
| Flow rate | 1.0 mL/min | |
| Injection volume | 5 µL | |
| Detection wavelength | 204 nm, 262 nm | |
| Column temperature | 40 °C | |
| Dilution solution | ACN/H₂O (1:1, v:v)^{∗} | |

| | | |
|---|---|---|
| *: add 10% DMSO in preparation to help dissolution. | | |

**Table 8-2. HPLC test parameters for solubility**

| **Parameter** | **Set value** | |
|---|---|---|
| Chromatography column | YMC Triart C18, 150 × 4.6 mm, 3.0 µm | |
| Mobile phase | A: 0.1% TFA in water | |
| | B: 0.1% TFA in acetonitrile | |
| Gradient | Time (min) | %Mobile phase B |
| | 0.0 | 5 |
| | 6.0 | 80 |
| | 8.0 | 80 |
| | 8.1 | 5 |
| | 10.0 | 5 |
| Time | 10 min | |
| Flow rate | 1.0 mL/min | |
| Injection volume | 5 µL | |
| Detection wavelength | 206 nm | |
| Column temperature | 37°C | |
| Dilution solution | ACN/H₂O (1:1, v:v) | |

### Hydraulic press

The Hydraulic press experiment is carried out on an SYP-5BS manual press, and the pressure put on to the sample is about 350 MPa.

### BRIEF DESCRIPTION OF THE DRAWINGS

FIG. 1 shows an XRPD pattern of crystal form A.
FIG. 2 shows an XRPD pattern of crystal form B.
FIG. 3 shows an XRPD pattern of crystal form C.
FIG. 4 shows an XRPD pattern of crystal form D.
FIG. 5 shows an XRPD pattern of crystal form E.
FIG. 6 shows a TGA profile of crystal form A.
FIG. 7 shows a DSC profile of crystal form A.
FIG. 8 shows a TGA profile of crystal form B.
FIG. 9 shows a DSC profile of crystal form B.
FIG. 10 shows a TGA profile of crystal form C.
FIG. 11 shows a DSC profile of crystal form C.
FIG. 12 shows a TGA profile of crystal form D.
FIG. 13 shows a DSC profile of crystal form D.
FIG. 14 shows a TGA profile of crystal form E.
FIG. 15 shows a DSC profile of crystal form E.
FIG. 16 shows a three-dimensional ellipsoid plot of crystal form C.

### DETAILED DESCRIPTION

The present disclosure is described in detail below by way of examples. However, this is by no means disadvantageously limiting the scope of the present disclosure. The compounds of the present disclosure can be prepared by a variety of synthetic methods well known to those skilled in the art, including the specific embodiments listed below, embodiments formed by combinations thereof with other chemical synthetic methods, and equivalents thereof known to those skilled in the art. Preferred embodiments include, but are not limited to, the examples of the present disclosure. It will be apparent to those skilled in the art that various changes and modifications can be made to the specific embodiments of the present invention without departing from the spirit and scope of the present invention.

### Example 1. Preparation of the Compound of Formula (I)

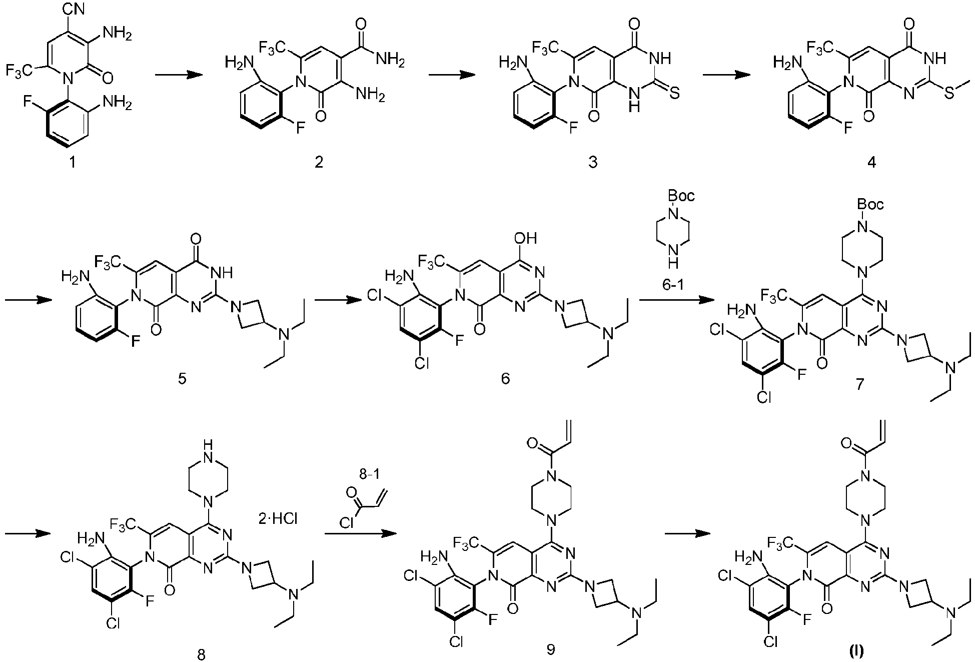

### Step 1: Synthesis of compound 2

To a solution of **compound 1** (1500 g, 4.49 mol) in ethanol (10.5 L) and water (4.5 L) was added potassium carbonate (1.24 kg, 8.97 mol), and then the temperature was raised to 50 °C. Hydrogen peroxide (1.78 kg, 15.71 mol, 30% purity) was slowly and directly added dropwise, with the temperature controlled at 50-70 °C. After the dropwise addition, the mixture was stirred at 70 °C for another hour. The temperature was reduced to 30 °C, and an aqueous solution of sodium sulfite (3.0 L) was added. The mixture was then stirred at 30 °C for 1 h. Tap water (30 L) was added, and the mixture was stirred at 20 °C for 1 h. The solid was collected by filtration and dried to give **compound 2.** LCMS (ESI) m/z: 331.0 (M+1).

### Step 2: Synthesis of compound 3

To a solution of **compound 2** (300 g, 0.84 mol) in acetonitrile (3.0 L) were added potassium hydroxide (189.98 g, 3.39 mol) and carbon disulfide (193.35 g, 2.54 mol). The reaction mixture was stirred at 25 °C for 2 h. Tap water (7.5 L) was added, and the pH was adjusted to 1 with 2 M hydrochloric acid (1.8 L). The precipitate was collected by filtration, washed with water, and dried to give **compound 3.** LCMS (ESI) m/z: 373.0 (M+1).

### Step 3: Synthesis of compound 4

To a solution of **compound 3** (1000 g, 2.69 mol) in dimethyl carbonate (10.0 L) were added potassium carbonate (816.69 g, 5.91 mol) and tetrabutylammonium bromide (86.59 g, 268.60 mmol). The reaction mixture was stirred at an internal temperature of 85-90 °C for 16 h, cooled to 30 °C, and filtered, and the filter cake was then diluted in 14 L of water. The mixture was stirred at 10 °C for 3 h and then adjusted to pH 2 with 6 M hydrochloric acid. The solid was collected by filtration and dried to give a crude product (1085 g). The crude product was stirred with a mixed solvent of ethyl acetate (3254 mL) and petroleum ether (4881 mL) at 20 °C for 10 h. The solid was collected by filtration and dried to give **compound 4.** LCMS (ESI) m/z: 387.0 (M+1).

### Step 4: Synthesis of compound 5

To a solution of N,N-diethylazetidin-3-amine (159.70 g, 1.25 mol) in n-butanol (3510 mL) was added **Compound 4** (390 g, 0.96 mol). The reaction mixture was heated to 120 °C, stirred for 15 h, and cooled to 30 °C. Water (4.0 L) was added, and layers formed. The aqueous phase was extracted twice with ethyl acetate (1 L × 2), and the organic phases were combined and concentrated under reduced pressure. To the resulting residue were added ethyl acetate (4 L) and water (3.0 L), and the pH was adjusted to 2 with 6 M hydrochloric acid. The organic phase was separated and discarded. The aqueous phase was adjusted to pH 8 with potassium carbonate (250 g) and then extracted three times with ethyl acetate (2.5 L × 3). The extract was dried over anhydrous sodium sulfate, filtered, and concentrated to dryness to give **compound 5.** LCMS (ESI) m/z: 467.1 (M+1).

### Step 5: Synthesis of compound 6

To a solution of **compound 5** (830 g, 1.69 mmol) in acetonitrile (2.52 L) were added TFA (771.02 g, 6.76 mmol) and NCS (1451.47 g, 3.38 mmol). The reaction mixture was heated to 60 °C, stirred for 2 h, and quenched by adding a saturated aqueous solution of sodium sulfite (1.6 L). Then 1 M sodium hydroxide solution (7 L) was added dropwise, and the mixture was extracted three times with ethyl acetate (4 L × 3). The extract was washed once with saturated brine (1 L), dried, filtered, and concentrated to give **compound 6.** LCMS (ESI) m/z: 535.0 (M+1).

### Step 6: Synthesis of compound 7

To a solution of **Compound 6** (400 g, 0.69 mol) in N,N-dimethylacetamide (3.2 L) were added N,N-diisopropylethylamine (135.05 g, 1.04 mol), **compound 6-1** (142.72 g, 0.76 mol) and PyBrOP (357.23 g, 0.76 mmol). The reaction mixture was stirred at 20 °C for 3 h. To the reaction mixture was added purified water (9.6 L). The mixture was then filtered, and the filter cake was dried to give **compound 7.** LCMS (ESI) m/z: 703.1 (M+1).

### Step 7: Synthesis of compound 8

To a solution of **compound 7** (600 g, 0.85 mol) in ethyl acetate (1.8 L) was added a 4 M solution of hydrochloric acid in methanol (1.8 L, 7.20 mol). The reaction mixture was stirred at 15 °C for 12 h and filtered. The filter cake was triturated with ethyl acetate (2 L) at 15 °C for 2 h. The mixture was filtered, and the filter cake was dried to give **compound 8.** LCMS (ESI) m/z: 603.1 (M+1).

### Step 8: Synthesis of the compound of formula (I)

To a solution of **compound 8** (360 g, 0.53 mmol) in 2-methyltetrahydrofuran (3.6 L) were added water (7.2 L) and potassium carbonate (146.70 g, 1.06 mol). The reaction mixture was cooled to 15 °C, and then **compound 8-1** (57.64 g, 0.64 mol) was added dropwise. The reaction mixture was stirred at 15 °C for 15 min and extracted twice with 2-methyltetrahydrofuran (0.5 L × 2). The organic phases were combined, washed once with saturated brine (0.5 L), dried over anhydrous sodium sulfate, and filtered. The filtrate was concentrated under reduced pressure to give a crude product. The crude product was stirred with a mixed solvent of methanol/water = 1/2 (2.8 L) at 40 °C for 12 h. The mixture was then filtered, and the filter cake was dried and then added to isopropanol (1.5 L) to form a suspension. The suspension was heated to 80 °C and stirred at 80 °C for 2 h. Then n-heptane (1.0 L) was added. The reaction mixture was stirred at 80 °C for 12 h, cooled to 25 °C, stirred at 25 °C for another 12 h, and then filtered. The filter cake was dried to give **the compound of formula (I).** LCMS (ESI) m/z: 656.9 (M+1); ¹H NMR (400 MHz, DMSO-*d₆*) δ 7.68 (d, J=7.58 Hz, 1 H) 7.00 (s, 1 H) 6.82 (dd, J=16.69, 10.45 Hz, 1 H) 6.10 - 6.22 (m, 3 H) 5.69 - 5.77 (m, 1 H) 4.09 - 4.22 (m, 2 H) 3.85 - 3.94 (m, 2 H) 3.79 (br s, 2 H) 3.71 (br s, 6 H) 3.60 - 3.65 (m, 1 H) 2.51 - 2.57 (m, 4 H) 0.91 - 0.99 (m, 6 H).

### Example 2. Preparation of Crystal Forms of the Compound of Formula (I)

### Preparation of crystal form A of the compound of formula (I):

The compound of formula (I) (10 g) was dissolved in 130 mL of isopropanol. The solution was heated to 100 °C, stirred at 100 °C for 1 h, then naturally cooled to 10 °C, stirred at 10 °C for 9 h, and filtered, and crystal form A was obtained.

### Preparation of crystal form B of the compound of formula (I):

The compound of formula (I) (19.0 mg) was dissolved in 1.0 mL of tetrahydrofuran. To the solution was added dropwise 3.6 mL of anti-solvent water, and crystal form B was obtained.

### Preparation of crystal form C of the compound of formula (I):

The compound of formula (I) (19.7 mg) was dissolved in 1.0 mL of acetonitrile. To the solution was added dropwise 3.7 mL of anti-solvent water, and crystal form C was obtained.

### Preparation of crystal form D of the compound of formula (I):

The compound of formula (I) (14.7 mg) was dissolved in 1.0 mL of ethyl acetate. To the solution was added dropwise 5.8 mL of anti-solvent n-heptane, and crystal form D was obtained.

The compound of formula (I) (22.0 mg) was added to 0.6 mL of isopropyl acetate/m-xylene (v/v, 1:3) to form a suspension. The suspension was equilibrated at 50 °C for 2 h, then filtered, cooled to 5 °C at a rate of 0.1 °C/min, and then volatilized at room temperature for about 1 month, and crystal form D was obtained.

The compound of formula (I) (32.2 mg) was added to 0.5 mL of acetonitrile/m-xylene (v/v, 1:5) to form a suspension. The suspension was equilibrated at 50 °C for 2 h, then cooled to 5 °C at a rate of 0.1 °C/min, then heated to 50 °C at a rate of 0.1 °C/min, then cooled to 5 °C at a rate of 0.1 °C/min, and volatilized at room temperature for about 1 month, and crystal form D was obtained.

### Preparation of crystal form E of the compound of formula (I):

The compound of formula (I) (15.7 mg) was dissolved in 1.0 mL of 2-methyltetrahydrofuran. To the solution was added dropwise 3.8 mL of anti-solvent n-heptane, and crystal form E was obtained.

### Example 3. Pressure Stability of Crystal Form C of the Compound of Formula (I)

Crystal form C of the compound of formula (I) was added to a circular mold (6 mm in diameter) and pressurized to about 350 MPa. A small piece of the sheet-like sample was directly taken for XRPD analysis. The results showed that crystal form C did not change after being pressed (under a pressure of about 350 MPa). Conclusion: Crystal form C of the compound of formula (I) has good pressure stability.

### Example 4. Tests for Solid Stability of Crystal Form C of the Compound of Formula (I)

To evaluate the solid stability of crystal form C of the compound of formula (I), crystal form C of the compound of formula (I) was subjected to stability testing under affecting factor conditions (high temperature, high humidity, and light), 60 °C/75%RH conditions, and 40 °C/75%RH conditions. Crystal form C was placed under a high-temperature condition (60 °C, closed) and a high-humidity condition (92.5% RH, wrapped in a sealing film, with 5 small holes made) for 1 week and 2 weeks, placed in a closed container in visible light and ultraviolet light (a light-shielding control group was set at the same time, and the sample was wrapped in tinfoil) according to the ICH conditions (visible light illumination reached 1.2E+06 Lux·hrs, and ultraviolet light illumination reached 200 W·hrs/m2), and meanwhile placed under 60 °C/75%RH conditions (wrapped in a sealing film, with 5 small holes made) for 1 month and 2 months and under 40 °C/75%RH conditions (wrapped in a sealing film, with 5 small holes made) for 1 month, 2 months, and 3 months. All the stability samples were tested for crystal form change by XRPD analysis and were analyzed by HPLC.

Experimental results: see Table 9.

**Table 9. A summary of the conditions of the solid stability experiments for crystal form C of the compound of formula (I)**

| Stability condition | Point taking condition | Purity (%)* | Relative purity# | Crystal form change |
|---|---|---|---|---|
| Starting sample | -- | 97.85 | -- | Unchanged |
| 60 °C | 1 week | 97.99 | 100.1% | Unchanged |
| | 2 weeks | 97.98 | 100.1% | Unchanged |
| 92.5%RH | 1 week | 98.03 | 100.2% | Unchanged |
| | 2 weeks | 98.00 | 100.2% | Unchanged |
| Visible + ultraviolet (ICH conditions) | Visible light illumination reaches 1.2E+06 Lux·hrs Ultraviolet illumination reaches 300 W·hrs/m² | 96.62 | 98.7% | Unchanged |
| Light-shielding control group | Simultaneously with visible + ultraviolet | 98.00 | 100.2% | Unchanged |
| 60 °C/75%RH | 1 month | 97.92 | 100.1% | Unchanged |
| | 2 months | 97.98 | 100.1% | Unchanged |
| 40 °C/75%RH | 1 month | 98.00 | 100.2% | Unchanged |
| | 2 months | 97.92 | 100.1% | Unchanged |
| | 3 months | 98.02 | 100.2% | Unchanged |

| | | | | |
|---|---|---|---|---|
| *: Chromatographic peaks with a peak area of less than 0.05% were not integrated; #: Relative purity is the ratio of stability sample purity to starting sample purity. **Conclusion:** Crystal form C of the compound of formula (I) has good solid stability. | | | | |

### Example 5. Confirmation of Steric Configuration of the Compound of Formula (I)

Preparation of a crystal of the compound of formula (I): the crystal of the compound of formula (I) was obtained by 10 days of room-temperature growth under an ethanol condition using the solvent volatilization method. The ellipsoid plot of the three-dimensional structure of the compound of formula (I) is shown in FIG. 16: the compound is in an S configuration. The structural data and parameters of the crystal of the compound of formula (I) are shown in Tables 10-1, 10-2, 10-3, 10-4, 10-5, and 10-6.

**Table 10-1. The structural data and measurement parameters of the crystal of the compound of formula (I)**

| | |
|---|---|
| Empirical formula | C₂₈H₃₀Cl₂F₄N₈O₂ |
| Molecular weight | 657.5 |
| Temperature | 173(2) K |
| Wavelength | 1.54178 Å |
| Crystal system | Monoclinic |
| Space group | P2₁ |
| Unit cell size | a = 7.59450(10) Å alpha= 90°. |
| | b = 13.3853(2) Å beta= 93.5820(10)°. |
| | c = 15.0473(2) Å gama = 90°. |
| Volume | 1526.64(4) Å³ |
| Z | 2 |
| Calculating density | 1.430 Mg/m³ |
| Absorption coefficient | 2.489 mm⁻¹ |
| F(000) | 680 |
| Dimensions of crystal | 0.180 × 0.160 × 0.140 mm³ |
| Angle range for data collection | 2.942 to 68.255°. |
| Limiting index | -9<=h<=9, -16<=k<=15, -18<=1<=18 |
| Reflection collection | 22503 |
| Uniqueness | 5523 [R(int) = 0.0380] |
| Angular integrity = 25.242 | 99.90% |
| Processing method | F2 full matrix least square method |
| Data/limits/parameters | 5523 /4/399 |
| Goodness of Fit on F2 | 1.045 |
| Final R index [I>2sigma(I)] | R1 = 0.0471, wR2 = 0.1275 |
| R index (all data) | R1 = 0.0515, wR2 = 0.1328 |
| Absolute structural parameter | 0.050(7) |
| Extinction index | n/a |
| Maximum diffraction peak and hole | 0.540 and -0.435 e.Å⁻³ |

**Table 10-2. The atomic coordinates (× 10⁴) and equivalent isotropic displacement parameters (Å² × 10³) of the crystal of the compound of formula (I)**

| / | **x** | **y** | **z** | **U(eq)** |
|---|---|---|---|---|
| C1(1) | 9560(2) | 4387(1) | 9718(1) | 60(1) |
| C(1) | 7014(6) | 5605(3) | 7586(3) | 31(1) |
| O(1) | 8307(4) | 5207(3) | 6011(2) | 39(1) |
| N(1) | 7264(5) | 3972(2) | 8108(2) | 23(1) |
| F(1) | 6781(6) | 7294(3) | 7017(3) | 78(1) |
| Cl(2) | 9242(2) | 8151(1) | 8453(1) | 58(1) |
| C(2) | 7678(6) | 4944(3) | 8208(3) | 36(1) |
| O(2) | -4285(5) | 2232(3) | 4500(3) | 49(1) |
| F(2) | 1547(4) | 5312(3) | 7683(2) | 64(1) |
| N(2) | 5891(5) | 5267(3) | 6838(2) | 30(1) |
| F(4) | 3567(4) | 6414(3) | 7957(2) | 57(1) |
| C(4) | 9226(7) | 6247(4) | 9003(3) | 39(1) |
| N(4) | 6198(5) | 4800(3) | 4476(3) | 32(1) |
| F(3) | 3906(5) | 4926(3) | 8463(2) | 61(1) |
| C(3) | 8754(6) | 5243(4) | 8935(3) | 41(1) |
| N(3) | 3382(5) | 4235(3) | 3830(2) | 32(1) |
| N(5) | 1308(5) | 3573(3) | 4737(2) | 33(1) |
| C(5) | 8580(7) | 6911(4) | 8368(3) | 38(1) |
| N(8) | 5991(7) | 6365(4) | 1402(3) | 50(1) |
| C(8) | 5518(5) | 4798(3) | 5274(3) | 29(1) |
| N(7) | 5659(6) | 4684(3) | 2967(3) | 42(1) |
| C(7) | 6708(6) | 5105(3) | 6046(3) | 32(1) |
| N(6) | -1323(5) | 2070(3) | 4777(3) | 40(1) |
| C(6) | 7439(6) | 6629(4) | 7647(3) | 34(1) |
| C(9) | 3781(6) | 4526(3) | 5410(3) | 30(1) |
| C(10) | 3047(6) | 4773(3) | 6229(3) | 32(1) |
| C(12) | 3282(6) | 5461(5) | 7753(3) | 44(1) |
| C(11) | 4065(6) | 5143(3) | 6910(3) | 32(1) |
| C(13) | 2812(6) | 4103(3) | 4646(3) | 30(1) |
| C(14) | 5058(6) | 4583(3) | 3799(3) | 34(1) |
| C(15) | 910(6) | 3085(4) | 5572(3) | 35(1) |
| C(16) | 241(7) | 2035(4) | 5391(4) | 42(1) |
| C(17) | -127(6) | 3517(4) | 4026(3) | 36(1) |
| C(18) | -894(7) | 2468(4) | 3913(3) | 43(1) |
| C(19) | -3003(7) | 2065(4) | 5029(4) | 40(1) |
| C(20) | -3252(9) | 1819(5) | 5981(4) | 55(2) |
| C(21) | -4103(15) | 2305(6) | 6510(6) | 101(3) |
| C(22) | 4644(8) | 4863(5) | 2123(3) | 51(1) |
| C(23) | 6278(7) | 5385(5) | 1775(4) | 46(1) |
| C(24) | 7109(7) | 5338(5) | 2744(3) | 46(1) |
| C(25) | 4883(9) | 6286(5) | 557(4) | 60(2) |
| C(26) | 4123(15) | 7236(8) | 273(6) | 99(3) |
| C(27) | 7671(10) | 6854(6) | 1226(5) | 68(2) |
| C(28) | 8496(19) | 7409(10) | 1957(7) | 132(5) |

**Table 10-3. The bond lengths (Å) and bond angles [°] of the crystal of the compound of formula (I)**

| **Bond length** | | **Bond angle** | | **Bond angle** | |
|---|---|---|---|---|---|
| C1(1)-C(3) | 1.727(5) | C(2)-C(1)-C(6) | 120.9(4) | N(5)-C(17)-H(17A) | 109.1 |
| C(1)-C(2) | 1.362(6) | C(2)-C(1)-N(2) | 120.5(4) | C(18)-C(17)-H(17A) | 109.1 |
| C(1)-C(6) | 1.410(7) | C(6)-C(1)-N(2) | 118.6(4) | N(5)-C(17)-H(17B) | 109.1 |
| C(1)-N(2) | 1.442(6) | C(2)-N(1)-H(1A) | 120 | C(18)-C(17)-H(17B) | 109.1 |
| O(1)-C(7) | 1.226(5) | C(2)-N(1)-H(1B) | 120 | H(17A)-C(17)-H(17B) | 107.8 |
| N(1)-C(2) | 1.344(6) | H(1A)-N(1)-H(1B) | 120 | N(6)-C(18)-C(17) | 110.0(4) |
| N(1)-H(1A) | 0.8800 | N(1)-C(2)-C(1) | 118.6(4) | N(6)-C(18)-H(18A) | 109.7 |
| N(1)-H(1B) | 0.8800 | N(1)-C(2)-C(3) | 119.4(4) | C(17)-C(18)-H(18A) | 109.7 |
| F(1)-C(6) | 1.372(6) | C(1)-C(2)-C(3) | 122.0(4) | N(6)-C(18)-H(18B) | 109.7 |
| Cl(2)-C(5) | 1.738(5) | C(7)-N(2)-C(11) | 122.7(4) | C(17)-C(18)-H(18B) | 109.7 |
| C(2)-C(3) | 1.384(7) | C(7)-N(2)-C(1) | 116.3(3) | H(18A)-C(18)-H(18B) | 108.2 |
| O(2)-C(19) | 1.239(6) | C(11)-N(2)-C(1) | 121.0(4) | O(2)-C(19)-N(6) | 122.4(5) |
| F(2)-C(12) | 1.331(6) | C(5)-C(4)-C(3) | 119.6(5) | O(2)-C(19)-C(20) | 121.0(5) |
| N(2)-C(7) | 1.394(6) | C(5)-C(4)-H(4) | 120.2 | N(6)-C(19)-C(20) | 116.6(5) |
| N(2)-C(11) | 1.407(6) | C(3)-C(4)-H(4) | 120.2 | C(21)-C(20)-C(19) | 127.1(7) |
| F(4)-C(12) | 1.326(7) | C(14)-N(4)-C(8) | 114.8(4) | C(21)-C(20)-H(20) | 116.5 |
| C(4)-C(5) | 1.374(7) | C(2)-C(3)-C(4) | 118.3(4) | C(19)-C(20)-H(20) | 116.5 |
| C(4)-C(3) | 1.393(8) | C(2)-C(3)-Cl(1) | 121.0(4) | C(20)-C(21)-H(21A) | 120 |
| C(4)-H(4) | 0.9500 | C(4)-C(3)-Cl(1) | 120.7(4) | C(20)-C(21)-H(21B) | 120 |
| N(4)-C(14) | 1.328(6) | C(13)-N(3)-C(14) | 115.7(4) | H(21A)-C(21)-H(21B) | 120 |
| N(4)-C(8) | 1.336(6) | C(13)-N(5)-C(15) | 122.7(4) | N(7)-C(22)-C(23) | 88.8(4) |
| F(3)-C(12) | 1.347(7) | C(13)-N(5)-C(17) | 122.6(4) | N(7)-C(22)-H(22A) | 113.9 |
| N(3)-C(13) | 1.339(6) | C(15)-N(5)-C(17) | 114.5(3) | C(23)-C(22)-H(22A) | 113.9 |
| N(3)-C(14) | 1.359(6) | C(4)-C(5)-C(6) | 122.9(5) | N(7)-C(22)-H(22B) | 113.9 |
| N(5)-C(13) | 1.359(6) | C(4)-C(5)-Cl(2) | 118.5(4) | C(23)-C(22)-H(22B) | 113.9 |
| N(5)-C(15) | 1.463(6) | C(6)-C(5)-Cl(2) | 118.5(4) | H(22A)-C(22)-H(22B) | 111.1 |
| N(5)-C(17) | 1.482(6) | C(23)-N(8)-C(27) | 111.3(5) | N(8)-C(23)-C(22) | 116.1(5) |
| C(5)-C(6) | 1.397(7) | C(23)-N(8)-C(25) | 109.5(5) | N(8)-C(23)-C(24) | 116.5(5) |
| N(8)-C(23) | 1.438(8) | C(27)-N(8)-C(25) | 109.2(5) | C(22)-C(23)-C(24) | 87.2(4) |
| N(8)-C(27) | 1.473(8) | N(4)-C(8)-C(9) | 123.7(4) | N(8)-C(23)-H(23) | 111.7 |
| N(8)-C(25) | 1.484(7) | N(4)-C(8)-C(7) | 116.9(4) | C(22)-C(23)-H(23) | 111.7 |
| C(8)-C(9) | 1.396(6) | C(9)-C(8)-C(7) | 119.5(4) | C(24)-C(23)-H(23) | 111.7 |
| C(8)-C(7) | 1.484(6) | C(14)-N(7)-C(24) | 124.7(4) | N(7)-C(24)-C(23) | 88.4(4) |
| N(7)-C(14) | 1.365(6) | C(14)-N(7)-C(22) | 128.6(4) | N(7)-C(24)-H(24A) | 113.9 |
| N(7)-C(24) | 1.462(7) | C(24)-N(7)-C(22) | 93.8(4) | C(23)-C(24)-H(24A) | 113.9 |
| N(7)-C(22) | 1.463(7) | O(1)-C(7)-N(2) | 120.9(4) | N(7)-C(24)-H(24B) | 113.9 |
| N(6)-C(19) | 1.354(7) | O(1)-C(7)-C(8) | 123.6(4) | C(23)-C(24)-H(24B) | 113.9 |
| N(6)-C(16) | 1.460(6) | N(2)-C(7)-C(8) | 115.5(4) | H(24A)-C(24)-H(24B) | 111.1 |
| N(6)-C(18) | 1.460(7) | C(19)-N(6)-C(16) | 124.5(4) | C(26)-C(25)-N(8) | 112.6(6) |
| C(9)-C(10) | 1.423(6) | C(19)-N(6)-C(18) | 121.2(4) | C(26)-C(25)-H(25A) | 109.1 |
| C(9)-C(13) | 1.441(6) | C(16)-N(6)-C(18) | 111.0(4) | N(8)-C(25)-H(25A) | 109.1 |
| C(10)-C(11) | 1.339(6) | F(1)-C(6)-C(5) | 122.9(5) | C(26)-C(25)-H(25B) | 109.1 |
| C(10)-H(10) | 0.9500 | F(1)-C(6)-C(1) | 120.9(4) | N(8)-C(25)-H(25B) | 109.1 |
| C(12)-C(11) | 1.497(7) | C(5)-C(6)-C(1) | 116.2(4) | H(25A)-C(25)-H(25B) | 107.8 |
| C(15)-C(16) | 1.513(7) | C(8)-C(9)-C(10) | 119.4(4) | C(25)-C(26)-H(26A) | 109.5 |
| C(15)-H(15A) | 0.9900 | C(8)-C(9)-C(13) | 115.2(4) | C(25)-C(26)-H(26B) | 109.5 |
| C(15)-H(15B) | 0.9900 | C(10)-C(9)-C(13) | 125.0(4) | H(26A)-C(26)-H(26B) | 109.5 |
| C(16)-H(16A) | 0.9900 | C(11)-C(10)-C(9) | 120.6(4) | C(25)-C(26)-H(26C) | 109.5 |
| C(16)-H(16B) | 0.9900 | C(11)-C(10)-H(10) | 119.7 | H(26A)-C(26)-H(26C) | 109.5 |
| C(17)-C(18) | 1.526(7) | C(9)-C(10)-H(10) | 119.7 | H(26B)-C(26)-H(26C) | 109.5 |
| C(17)-H(17A) | 0.9900 | F(4)-C(12)-F(2) | 108.0(5) | C(28)-C(27)-N(8) | 115.7(6) |
| C(17)-H(17B) | 0.9900 | F(4)-C(12)-F(3) | 106.5(4) | C(28)-C(27)-H(27A) | 108.4 |
| C(18)-H(18A) | 0.9900 | F(2)-C(12)-F(3) | 106.3(4) | N(8)-C(27)-H(27A) | 108.4 |
| C(18)-H(18B) | 0.9900 | F(4)-C(12)-C(11) | 113.7(4) | C(28)-C(27)-H(27B) | 108.4 |
| C(19)-C(20) | 1.493(8) | F(2)-C(12)-C(11) | 109.5(4) | N(8)-C(27)-H(27B) | 108.4 |
| C(20)-C(21) | 1.240(10) | F(3)-C(12)-C(11) | 112.5(4) | H(27A)-C(27)-H(27B) | 107.4 |
| C(20)-H(20) | 0.9500 | C(10)-C(11)-N(2) | 120.6(4) | C(27)-C(28)-H(28A) | 109.5 |
| C(21)-H(21A) | 0.9500 | C(10)-C(11)-C(12) | 120.9(4) | C(27)-C(28)-H(28B) | 109.5 |
| C(21)-H(21B) | 0.9500 | N(2)-C(11)-C(12) | 118.5(4) | H(28A)-C(28)-H(28B) | 109.5 |
| C(22)-C(23) | 1.543(8) | N(3)-C(13)-N(5) | 118.9(4) | C(27)-C(28)-H(28C) | 109.5 |
| C(22)-H(22A) | 0.9900 | N(3)-C(13)-C(9) | 120.1(4) | H(28A)-C(28)-H(28C) | 109.5 |
| C(22)-H(22B) | 0.9900 | N(5)-C(13)-C(9) | 121.0(4) | H(28B)-C(28)-H(28C) | 109.5 |
| C(23)-C(24) | 1.554(7) | N(4)-C(14)-N(3) | 128.0(4) | | |
| C(23)-H(23) | 1.0000 | N(4)-C(14)-N(7) | 116.3(4) | | |
| C(24)-H(24A) | 0.9900 | N(3)-C(14)-N(7) | 115.7(4) | | |
| C(24)-H(24B) | 0.9900 | N(5)-C(15)-C(16) | 110.2(4) | | |
| C(25)-C(26) | 1.450(11) | N(5)-C(15)-H(15A) | 109.6 | | |
| C(25)-H(25A) | 0.9900 | C(16)-C(15)-H(15A) | 109.6 | | |
| C(25)-H(25B) | 0.9900 | N(5)-C(15)-H(15B) | 109.6 | | |
| C(26)-H(26A) | 0.9800 | C(16)-C(15)-H(15B) | 109.6 | | |
| C(26)-H(26B) | 0.9800 | H(15A)-C(15)-H(15B) | 108.1 | | |
| C(26)-H(26C) | 0.9800 | N(6)-C(16)-C(15) | 109.5(4) | | |
| C(27)-C(28) | 1.438(12) | N(6)-C(16)-H(16A) | 109.8 | | |
| C(27)-H(27A) | 0.9900 | C(15)-C(16)-H(16A) | 109.8 | | |
| C(27)-H(27B) | 0.9900 | N(6)-C(16)-H(16B) | 109.8 | | |
| C(28)-H(28A) | 0.9800 | C(15)-C(16)-H(16B) | 109.8 | | |
| C(28)-H(28B) | 0.9800 | H(16A)-C(16)-H(16B) | 108.2 | | |
| C(28)-H(28C) | 0.9800 | N(5)-C(17)-C(18) | 112.6(4) | | |

**Table 10-4. Anisotropic displacement parameters (Å2 × 103) and anisotropic displacement factor indexes of the crystal of the compound of formula (I)**

| (calculation formula: -2π² [ h² a^{∗2}U¹¹ + ... + 2 h k a^{∗} b^{∗} U¹² ]) | | | | | | |
|---|---|---|---|---|---|---|
| | U11 | U22 | U33 | U23 | U13 | U12 |
| Cl(1) | 72(1) | 67(1) | 40(1) | 14(1) | -5(1) | 20(1) |
| C(1) | 30(2) | 30(2) | 34(2) | 2(2) | 5(2) | 2(2) |
| O(1) | 28(2) | 49(2) | 42(2) | -3(2) | 4(1) | -2(1) |
| N(1) | 34(2) | 16(1) | 20(2) | 8(1) | -1(1) | 2(1) |
| F(1) | 101(3) | 63(3) | 70(3) | 13(2) | -7(2) | 3(2) |
| Cl(2) | 77(1) | 47(1) | 47(1) | -11(1) | -12(1) | -15(1) |
| C(2) | 39(2) | 32(2) | 37(2) | 0(2) | 8(2) | -1(2) |
| O(2) | 39(2) | 49(2) | 58(2) | 7(2) | -7(2) | -3(2) |
| F(2) | 35(2) | 106(3) | 54(2) | -27(2) | 16(1) | -5(2) |
| N(2) | 30(2) | 28(2) | 33(2) | -1(1) | 5(1) | -2(2) |
| F(4) | 52(2) | 63(2) | 58(2) | -28(2) | 8(2) | 3(2) |
| C(4) | 37(2) | 57(3) | 23(2) | -3(2) | 1(2) | 5(2) |
| N(4) | 33(2) | 31(2) | 34(2) | 0(2) | 8(2) | 5(2) |
| F(3) | 57(2) | 90(3) | 36(2) | 7(2) | 11(1) | -4(2) |
| C(3) | 39(2) | 53(3) | 30(2) | 7(2) | 6(2) | 10(2) |
| N(3) | 39(2) | 28(2) | 31(2) | -2(1) | 6(2) | 0(2) |
| N(5) | 32(2) | 35(2) | 31(2) | 2(2) | 0(2) | -2(2) |
| C(5) | 39(3) | 40(3) | 35(2) | -5(2) | 4(2) | -2(2) |
| N(8) | 58(3) | 57(3) | 35(2) | 4(2) | 4(2) | -5(2) |
| C(8) | 30(2) | 24(2) | 34(2) | 0(2) | 2(2) | 3(2) |
| N(7) | 46(2) | 48(2) | 33(2) | 1(2) | 10(2) | -5(2) |
| C(7) | 33(2) | 24(2) | 40(2) | 0(2) | 9(2) | 0(2) |
| N(6) | 34(2) | 48(2) | 37(2) | 3(2) | -5(2) | -12(2) |
| C(6) | 34(2) | 37(2) | 30(2) | -2(2) | 3(2) | -1(2) |
| C(9) | 30(2) | 25(2) | 34(2) | -2(2) | 6(2) | 2(2) |
| C(10) | 30(2) | 30(2) | 35(2) | -4(2) | 6(2) | -4(2) |
| C(12) | 32(2) | 64(4) | 38(3) | -8(2) | 6(2) | -6(2) |
| C(11) | 32(2) | 28(2) | 37(2) | -2(2) | 6(2) | 0(2) |
| C(13) | 30(2) | 26(2) | 35(2) | -3(2) | 2(2) | 2(2) |
| C(14) | 42(2) | 25(2) | 37(2) | -1(2) | 11(2) | 4(2) |
| C(15) | 34(2) | 38(2) | 32(2) | 3(2) | 1(2) | -5(2) |
| C(16) | 38(3) | 43(3) | 44(3) | 10(2) | -8(2) | -7(2) |
| C(17) | 30(2) | 43(3) | 33(2) | 4(2) | -1(2) | 1(2) |
| C(18) | 41(3) | 53(3) | 33(3) | -1(2) | -5(2) | -6(2) |
| C(19) | 42(3) | 30(2) | 49(3) | -2(2) | 1(2) | -6(2) |
| C(20) | 66(4) | 47(3) | 55(3) | -2(3) | 9(3) | -18(3) |
| C(21) | 158(9) | 70(5) | 80(5) | 39(4) | 49(6) | 53(5) |
| C(22) | 59(3) | 59(3) | 34(3) | -4(2) | 4(2) | -16(3) |
| C(23) | 49(3) | 52(3) | 38(3) | 4(2) | 13(2) | 4(2) |
| C(24) | 40(3) | 56(3) | 43(3) | 10(2) | 9(2) | -2(2) |
| C(25) | 72(4) | 71(4) | 36(3) | -1(3) | -4(3) | -1(3) |
| C(26) | 131(8) | 101(7) | 62(5) | 0(4) | -21(5) | 34(6) |
| C(27) | 74(5) | 76(5) | 55(4) | 7(3) | 16(3) | -16(4) |
| C(28) | 181(11) | 138(9) | 77(6) | -2(6) | 17(6) | -105(9) |

**Table 10-5. The hydrogen coordinates (× 10⁴) and isotropic displacement parameters (Å² × 10³) of the crystal of the compound of formula (I)**

| | x | y | z | U(eq) |
|---|---|---|---|---|
| H(1A) | 6578 | 3776 | 7648 | 28 |
| H(1B) | 7679 | 3532 | 8502 | 28 |
| H(4) | 9990 | 6471 | 9487 | 47 |
| H(10) | 1823 | 4673 | 6294 | 38 |
| H(15A) | 5 | 3475 | 5867 | 42 |
| H(15B) | 1988 | 3061 | 5977 | 42 |
| H(16A) | 1171 | 1631 | 5130 | 51 |
| H(16B) | -55 | 1716 | 5956 | 51 |
| H(17A) | 335 | 3733 | 3456 | 43 |
| H(17B) | -1080 | 3984 | 4168 | 43 |
| H(18A) | -1972 | 2489 | 3508 | 51 |
| H(18B) | -27 | 2025 | 3645 | 51 |
| H(20) | -2699 | 1227 | 6205 | 66 |
| H(21A) | -4683 | 2904 | 6320 | 121 |
| H(21B) | -4174 | 2078 | 7105 | 121 |
| H(22A) | 3620 | 5313 | 2176 | 61 |
| H(22B) | 4296 | 4246 | 1796 | 61 |
| H(23) | 6914 | 4941 | 1367 | 55 |
| H(24A) | 8282 | 5013 | 2798 | 55 |
| H(24B) | 7128 | 5988 | 3058 | 55 |
| H(25A) | 5612 | 6026 | 85 | 72 |
| H(25B) | 3920 | 5801 | 636 | 72 |
| H(26A) | 5001 | 7626 | -28 | 148 |
| H(26B) | 3761 | 7605 | 793 | 148 |
| H(26C) | 3092 | 7118 | -139 | 148 |
| H(27A) | 8507 | 6334 | 1047 | 81 |
| H(27B) | 7460 | 7313 | 714 | 81 |
| H(28A) | 9209 | 7949 | 1725 | 198 |
| H(28B) | 9255 | 6963 | 2328 | 198 |
| H(28C) | 7586 | 7694 | 2315 | 198 |

**Table 10-6. The torsion angles [°] of the crystal of the compound of formula (I)**

| | | | |
|---|---|---|---|
| C(6)-C(1)-C(2)-N(1) | -178.5(4) | F(3)-C(12)-C(11)-N(2) | -63.8(6) |
| N(2)-C(1)-C(2)-N(1) | 0.0(6) | C(14)-N(3)-C(13)-N(5) | -166.7(4) |
| C(6)-C(1)-C(2)-C(3) | 1.7(7) | C(14)-N(3)-C(13)-C(9) | 13.8(6) |
| N(2)-C(1)-C(2)-C(3) | -179.8(4) | C(15)-N(5)-C(13)-N(3) | 157.5(4) |
| C(2)-C(1)-N(2)-C(7) | -97.1(5) | C(17)-N(5)-C(13)-N(3) | -28.0(6) |
| C(6)-C(1)-N(2)-C(7) | 81.4(5) | C(15)-N(5)-C(13)-C(9) | -23.0(7) |
| C(2)-C(1)-N(2)-C(11) | 84.9(5) | C(17)-N(5)-C(13)-C(9) | 151.5(4) |
| C(6)-C(1)-N(2)-C(11) | -96.6(5) | C(8)-C(9)-C(13)-N(3) | -18.0(6) |
| N(1)-C(2)-C(3)-C(4) | 177.2(4) | C(10)-C(9)-C(13)-N(3) | 155.1(4) |
| C(1)-C(2)-C(3)-C(4) | -3.0(7) | C(8)-C(9)-C(13)-N(5) | 162.5(4) |
| N(1)-C(2)-C(3)-Cl(1) | -0.4(6) | C(10)-C(9)-C(13)-N(5) | -24.4(7) |
| C(1)-C(2)-C(3)-Cl(1) | 179.4(4) | C(8)-N(4)-C(14)-N(3) | -9.6(7) |
| C(5)-C(4)-C(3)-C(2) | 1.8(7) | C(8)-N(4)-C(14)-N(7) | 173.1(4) |
| C(5)-C(4)-C(3)-Cl(1) | 179.4(4) | C(13)-N(3)-C(14)-N(4) | 0.5(7) |
| C(3)-C(4)-C(5)-C(6) | 0.6(7) | C(13)-N(3)-C(14)-N(7) | 177.8(4) |
| C(3)-C(4)-C(5)-Cl(2) | -178.2(4) | C(24)-N(7)-C(14)-N(4) | -24.4(7) |
| C(14)-N(4)-C(8)-C(9) | 4.2(6) | C(22)-N(7)-C(14)-N(4) | -155.3(5) |
| C(14)-N(4)-C(8)-C(7) | -175.8(4) | C(24)-N(7)-C(14)-N(3) | 158.0(5) |
| C(11)-N(2)-C(7)-O(1) | -179.0(4) | C(22)-N(7)-C(14)-N(3) | 27.1(7) |
| C(1)-N(2)-C(7)-O(1) | 3.1(6) | C(13)-N(5)-C(15)-C(16) | -134.6(4) |
| C(11)-N(2)-C(7)-C(8) | 0.4(6) | C(17)-N(5)-C(15)-C(16) | 50.4(5) |
| C(1)-N(2)-C(7)-C(8) | -177.5(4) | C(19)-N(6)-C(16)-C(15) | -95.7(6) |
| N(4)-C(8)-C(7)-O(1) | -11.6(6) | C(18)-N(6)-C(16)-C(15) | 63.8(6) |
| C(9)-C(8)-C(7)-O(1) | 168.4(4) | N(5)-C(15)-C(16)-N(6) | -57.9(5) |
| N(4)-C(8)-C(7)-N(2) | 169.1(4) | C(13)-N(5)-C(17)-C(18) | 138.2(5) |
| C(9)-C(8)-C(7)-N(2) | -10.9(6) | C(15)-N(5)-C(17)-C(18) | -46.9(5) |
| C(4)-C(5)-C(6)-F(1) | 179.4(5) | C(19)-N(6)-C(18)-C(17) | 101.3(5) |
| Cl(2)-C(5)-C(6)-F(1) | -1.8(7) | C(16)-N(6)-C(18)-C(17) | -59.0(6) |
| C(4)-C(5)-C(6)-C(1) | -1.9(7) | N(5)-C(17)-C(18)-N(6) | 49.6(5) |
| Cl(2)-C(5)-C(6)-C(1) | 176.9(3) | C(16)-N(6)-C(19)-O(2) | 170.0(5) |
| C(2)-C(1)-C(6)-F(1) | 179.5(4) | C(18)-N(6)-C(19)-O(2) | 12.4(8) |
| N(2)-C(1)-C(6)-F(1) | 0.9(7) | C(16)-N(6)-C(19)-C(20) | -11.6(7) |
| C(2)-C(1)-C(6)-C(5) | 0.7(7) | C(18)-N(6)-C(19)-C(20) | -169.1(5) |
| N(2)-C(1)-C(6)-C(5) | -177.8(4) | O(2)-C(19)-C(20)-C(21) | -53.0(11) |
| N(4)-C(8)-C(9)-C(10) | -164.9(4) | N(6)-C(19)-C(20)-C(21) | 128.6(9) |
| C(7)-C(8)-C(9)-C(10) | 15.2(6) | C(14)-N(7)-C(22)-C(23) | 152.2(5) |
| N(4)-C(8)-C(9)-C(13) | 8.6(6) | C(24)-N(7)-C(22)-C(23) | 10.7(4) |
| C(7)-C(8)-C(9)-C(13) | -171.4(4) | C(27)-N(8)-C(23)-C(22) | 170.8(5) |
| C(8)-C(9)-C(10)-C(11) | -8.7(7) | C(25)-N(8)-C(23)-C(22) | -68.3(6) |
| C(13)-C(9)-C(10)-C(ll) | 178.5(4) | C(27)-N(8)-C(23)-C(24) | 70.3(6) |
| C(9)-C(10)-C(11)-N(2) | -2.0(7) | C(25)-N(8)-C(23)-C(24) | -168.9(5) |
| C(9)-C(10)-C(11)-C(12) | 176.3(5) | N(7)-C(22)-C(23)-N(8) | -128.4(5) |
| C(7)-N(2)-C(11)-C(10) | 6.1(6) | N(7)-C(22)-C(23)-C(24) | -10.1(4) |
| C(1)-N(2)-C(11)-C(10) | -176.0(4) | C(14)-N(7)-C(24)-C(23) | -154.4(5) |
| C(7)-N(2)-C(11)-C(12) | -172.2(4) | C(22)-N(7)-C(24)-C(23) | -10.7(4) |
| C(1)-N(2)-C(11)-C(12) | 5.6(6) | N(8)-C(23)-C(24)-N(7) | 128.0(5) |
| F(4)-C(12)-C(11)-C(10) | -121.0(5) | C(22)-C(23)-C(24)-N(7) | 10.1(4) |
| F(2)-C(12)-C(11)-C(10) | -0.1(7) | C(23)-N(8)-C(25)-C(26) | 163.5(7) |
| F(3)-C(12)-C(11)-C(10) | 117.8(5) | C(27)-N(8)-C(25)-C(26) | -74.4(9) |
| F(4)-C(12)-C(11)-N(2) | 57.3(6) | C(23)-N(8)-C(27)-C(28) | -87.8(10) |
| F(2)-C(12)-C(11)-N(2) | 178.2(4) | C(25)-N(8)-C(27)-C(28) | 151.2(9) |

### Experimental Example 1. Cell Assay

### Objective

The effect of the compound on cell proliferation was examined on NCI-H358, MIA-PA-CA-2, A375, SW1463, and A427 cells.

The compound of formula (I) is a KRAS G12C selective inhibitor, and it has been proved that it can bind covalently with the GDP-bound KRAS G12C protein and block the exchange of KRAS protein GDP/GTP, so that the KRAS protein is maintained in GDP-bound form in which it is in an inactivated state, thereby inhibiting cell and tumor growth.

### Cells used in the assay

| **Name** | **Type** | **Species** | **Brand** | **Cat. No.** |
|---|---|---|---|---|
| NCI-H358 | Lung cancer | Human | Procell | CL-0400 |
| MIA-PA-CA-2 | Pancreatic cancer | Human | Cobioer | CBP60544 |
| A375 | Melanoma | Human | Procell | CL-0014 |
| SW1463 | Human rectal adenocarcinoma cell | Human | Shanghai Xin Yu | ZY-813 |
| A427 | Human lung cancer cell | Human | Cobioer | CBP60083 |

### Main reagents and consumables

| **Name** | **Brand** | **Cat. No.** |
|---|---|---|
| RPMI1640 medium | BI | 01-100-1ACS |
| DMEM medium | BI | 06-1055-57-1ACS |
| EMEM medium | Wisent | 320-005-CL |
| Leibovit2 sL-15 medium | BasalMedia | L620KJ |
| Fetal bovine serum | Biosera | FB-1058/500 |
| Horse serum | Gibco | 16050122 |
| DMSO | SinoPharm | 30072418 |
| 0.25% Trypsin-EDTA | BasalMedia | S310KJ |
| 96-well cell culture plate | COSTAR | 3610 |
| CellTiter-Glo | Promega | G7573 |
| P/S | Procell | PB180120 |

### Instruments and equipment

| **Name** | **Brand** | **Model** |
|---|---|---|
| Biosafety cabinet | Haier | HR40-IIA2 |
| Carbon dioxide incubator | Thermo | 3131 |
| EnVision | PerkinElmer | NA |

### Experimental method

### Conditions for cell culture

Medium for H358 cell line: 89% RPMI1640 medium + 10% fetal bovine serum + 1% P/S
Medium for MIA-PA-CA-2 cell line: 86.5% DMEM medium + 10% fetal bovine serum + 2.5% horse serum + 1% P/S
Medium for A375 cell line: 89% DMEM medium + 10% fetal bovine serum + 1% P/S
Medium for SW1463 cell line: 89% Leibovit2 sL-15 medium + 10% fetal bovine serum + 1% P/S
Medium for A427 cell line: 89% EMEM medium + 10% fetal bovine serum + 1% P/S

### Cell plating

1. The medium and pancreatin used in the process of cell passaging were pre-heated in a water bath at 37 °C.
2. The cells were rinsed with pancreatin and digested with pancreatin until the cells were detached. A culture solution was added, and the cells and the culture solution were well mixed to stop the digestion.
3. The cells were pipetted into a 15 mL centrifuge tube, 0.01 mL of cell suspension was pipetted onto a count plate, and cells were counted.
4. According to the cell densities shown in the table below, a proper amount of each type of cells was transferred to a 15 mL centrifuge tube and made up to a volume of 9 mL with a corresponding cell culture solution.

| Cell name | Cell density (per well) |
|---|---|
| H358 | 4000 |
| MIA-PA-CA-2 | 1000 |
| A375 | 2000 |
| SW1463 | 2000 |
| A427 | 3000 |

5. According to the microplate layout in the appendix, 150 µL of sterile water was added to the peripheral horizontal rows of wells in a 96-well microplate, and 80 µL of cell suspension was added to each of the other wells. Then the cell plate was placed in an incubator and incubated.

### Compound preparation

The compound of formula (I) was diluted from 10 mM to 2 mM with DMSO and then serially diluted 3-fold to obtain a total of 9 concentration gradients.

2 µL of each concentration gradient of the compound was pipetted into a 78 µL medium for corresponding cells, and the compound and the medium were well mixed as a compound solution.

3. According to the microplate layout in the appendix, 20 µL of the compound solution was pipetted into each cell culture plate to a final concentration of 10 µM-0.0015 µM, and the final concentration of DMSO was 0.5%. To the control group, 20 µL of cell culture medium containing 2.5% DMSO was added, and the cell plate was returned to the incubator and incubated.

### Plate reading

1. On the day of compound treatment, the day-0 test plate was removed from the incubator, 50 µL of Cell Titer Glo was added, and the plate was centrifuged at 1000 rpm for 10 s, shaken at room temperature for 10 min, then centrifuged again at 1000 rpm for 10 s, and read on Envision.
2. After the cells were incubated with the compound for 72 h, the MIA-PA-CA-2, A375 and A427 cell test plates were removed from the incubator. To each well of the 96-well plates, 50 µL of Cell Titer-Glo was added, and the plates were centrifuged at 1000 rpm for 10 s, shaken at room temperature for 10 min, then centrifuged again at 1000 rpm for 10 s, and read on Envision.
3. After the cells were incubated with the compound for 120 h, the NCI-H358 cell test plate was removed from the incubator. To each well of the 96-well plate, 50 µL of Cell Titer-Glo was added, and the plate was centrifuged at 1000 rpm for 10 s, shaken at room temperature for 10 min, then centrifuged again at 1000 rpm for 10 s, and read on Envision.
4. After the cells were incubated with the compound for 144 h, the SW1463 cell test plate was removed from the incubator. To each well of the 96-well plate, 50 µL of CellTiter-Glo was added, and the plate was centrifuged at 1000 rpm for 10 s, shaken at room temperature for 10 min, then centrifuged again at 1000 rpm for 10 s, and read on Envision.

### Experimental results and discussion

The experimental results show that the compound of formula (I) exhibited good inhibitory activity against the proliferation of the human non-small cell lung cancer cell NCI-H358, the pancreatic cancer cell MIA-PA-CA-2, and the human rectal adenocarcinoma cell SW1463. Meanwhile, the compound of formula (I) exhibited weak activity on the melanoma cell A375 and the human lung cancer cell A427, which indicates that the compound has poor inhibitory activity against wild-type KRAS and KRAS (G12D) proteins and the off-target risk is low. The IC₅₀ data are detailed in Table 11 below.

**Table 11**

| Cell line | IC₅₀ (nM) | | | | |
|---|---|---|---|---|---|
| | NCI-H358 | MIA-PA-CA-2 | A375 | SW1463 | A427 |
| Compound of formula (I) | 12.99±1.85 | 33.96±7.03 | 4639.5±89.8 | 112.41±32.66 | >6975 |

## Claims

1. A crystal form C of a compound of formula (I) 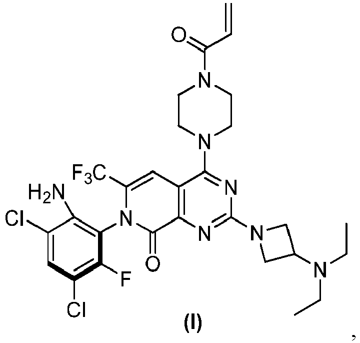 wherein an X-ray powder diffraction pattern of the crystal form C has characteristic diffraction peaks at the following 2Θ angles: 5.89±0.20°, 8.82±0.20°, and 17.71±0.20°.

2. The crystal form C according to claim 1, wherein the X-ray powder diffraction pattern of the crystal form C has characteristic diffraction peaks at the following 2θ angles: 5.89±0.20°, 8.82±0.20°, 13.19±0.20°, 14.81±0.20°, 17.71±0.20°, 18.72±0.20°, 21.58±0.20°, and 24.47±0.20°.

3. The crystal form C according to claim 2, wherein the X-ray powder diffraction pattern of the crystal form C has characteristic diffraction peaks at the following 2Θ angles: 5.89°, 8.82°, 11.50°, 12.58°, 13.19°, 14.42°, 14.81°, 17.71°, 18.72°, 20.70°, 21.58°, 23.51°, 24.47°, 25.36°, 26.58°, 27.09°, and 29.08°.

4. The crystal form C according to claim 2 or 3, wherein the XRPD pattern of the crystal form C is as shown in FIG. 3.

5. The crystal form C according to any one of claims 1-4, wherein a differential scanning calorimetry curve of the crystal form C has an endothermic peak starting point at 214.7±5 °C.

6. The crystal form C according to claim 5, wherein a DSC profile of the crystal form C is as shown in FIG. 11.

7. The crystal form C according to any one of claims 1-4, wherein a thermogravimetric analysis curve of the crystal form C shows a weight loss of 2.52% at 150.0±3 °C.

8. The crystal form C according to claim 7, wherein a TGA profile of the crystal form C is as shown in FIG. 10.

9. Use of the crystal form according to any one of claims 1-8 in the preparation of a medicament for treating cancer.

10. The use according to claim 9, wherein the cancer includes lung cancer, lymphoma, esophageal cancer, ovarian cancer, pancreatic cancer, rectal cancer, brain glioma, cervical cancer, urothelial cancer, stomach cancer, endometrial cancer, liver cancer, bile duct cancer, breast cancer, colon cancer, leukemia, and melanoma.
